Europäisches Patentamt

European Patent Office

Office européen des brevets

(11) Veröffentlichungsnummer: **0 121 002**
**A1**

(12) ## EUROPÄISCHE PATENTANMELDUNG

(21) Anmeldenummer: **83113197.4**

(22) Anmeldetag: **29.12.83**

(51) Int. Cl.³: **A 61 F 1/03**

(30) Priorität: **10.03.83 CH 1301/83**

(43) Veröffentlichungstag der Anmeldung:
**10.10.84 Patentblatt 84/41**

(84) Benannte Vertragsstaaten:
**AT DE FR GB IT NL SE**

(71) Anmelder: **Protek AG**
**Stadtbachstrasse 64**
**CH-3001 Bern(CH)**

(72) Erfinder: **Ganz, Reinhold, Prof. Dr.**
**Walchstrasse 10**
**CH-3073 Gümlingen(DE)**

(74) Vertreter: **Dipl.-Ing. H. Marsch Dipl.-Ing. K. Sparing**
**Dipl.-Phys.Dr. W.H. Röhl Patentanwälte**
**Rethelstrasse 123**
**D-4000 Düsseldorf(DE)**

(54) **Künstliche Hüftgelenkspfanne.**

(57) Zwei aus der Oberfläche der halbkugelförmigen Pfanne (1) herausragende Zapfen (6, 7) liegen mit ihren Achsen (8, 9) in einer Radialebene (10) der Pfannenschale (2); diese Ebene (10) bildet mit einer Mittelebene (11) der Pfanne (1) einen Winkel von 25°.

Zwischen den Zapfen (6, 7) ist eine Bohrung (15) zur Aufnahme einer Knochenschraube (16) vorgesehen.

Die Ausrichtung der Zapfenachsen (8, 9) in Richtung auf die Hauptbelastung (A) erhöht den festen Sitz der zementfrei zu verankernden Pfanne (1) entscheidend im Zusammenwirken mit der in gleicher Richtung verlaufenden Knochenschraube (16). Diese dient darüberhinaus vor allem einer sicheren Primärfixation.

Fig. 4

EP 0 121 002 A1

P. 5796/Wg/IS

- Protek AG, Stadtbachstr. 64, 3001 Bern/Schweiz

## Künstliche Hüftgelenkspfanne

Die Erfindung betrifft eine künstliche Hüftgelenkspfanne zur zementfreien Verankerung im Beckenknochen, mit einem kugelschalenförmigen Pfannenkörper, aus dem mit Verankerungsrippen versehene Zapfen hervorspringen.

Für eine zementfreie Verankerung haben sich Hüftgelenkspfannen der vorstehend genannten Art, die in ihrem konstruktiven Aufbau beispielsweise aus den FR-PS 1.552.585, FR-PS 2.197.561 und FR-PS 2.301.217 bekannt sind, bisher nicht bewährt; denn aufgrund der unvermeidlichen Bewegungen des Beckenknochens arbeitet sich die künstliche Hüftgelenkspfanne wegen ihrer Halbkugelform aus dem Knochen heraus (Kirschkern-Effekt), da vor allem in der ersten Zeit nach der Implantation wegen nicht ausreichender Primärverankerung die Fixation im Knochen ungenügend ist. Andererseits sind hablkugelförmige Pfannen den kegelstumpfförmigen vorzuziehen, weil sie bei gleicher "Grösse" ein kleineres Volumen besitzen, und das Bestreben dahingeht, den in das Körpergewebe einzusetzenden Fremdkörper so klein wie möglich zu halten.

Aufgabe der Erfindung ist es daher, eine halbkugelförmige Hüftgelenkspfanne zu schaffen, die im Beckenknochen so zu verankern ist, dass die Verankerung und vor allem ihre Primärfixation den Bewegungen des Beckens widersteht. Diese Aufgabe wird erfindungsgemäss dadurch gelöst, dass die durch die Achsen der Zapfen definierte Ebene mindestens im wesentlichen eine Radialebene der Pfannenschale ist, und dass in dieser Ebene zwischen den Zapfen durch den kugelschalenförmigen Pfannenkörper eine Bohrung zur Aufnahme einer Knochenschraube führt, wobei die Achse der Bohrung mindestens annähernd ebenfalls in der Radialebene der Zapfenachse liegt, und ferner durch eine Knochenschraube zur Fixierung der Pfanne im Beckenknochen.

Die Lagen der beiden Zapfenachsen in einer Radialebene - und nicht wie bei einer der bekannten Konstruktionen (FR-PS 1.552.585) in radialer Richtung der Pfannenschale - bewirken, dass beide Zapfenachsen in Richtung der Hauptbelastungen der Pfanne ausgerichtet werden können und daher aufgrund ihrer vorzugsweise zum Pfannenkörper hin, d.h. entgegen der Einschlagrichtung geneigten, in gewissem Umfang elastischen Verankerungsrippen eine besonders feste Verankerung ergeben. Eine solche Radialebene zeichnet sich dadurch aus, dass sie durch das Zentrum der Pfannenschale und damit bei in die Pfannenschale eingreifendem Gelenkkopf durch dessen Mittelpunkt verläuft.

Die verbesserte Primärfixation wird durch die in der gleichen Ebene liegenden Knochenschraube erreicht, deren Wirkung selbstverständlich nach dem Ein- oder Anwachsen von Gewebe nicht kleiner oder unwirksam wird, sondern sich zu derjenigen der Zapfen hinzuaddiert. Für eine Uebereinstimmung der Radialebene der Zapfenachsen mit der Hauptbelastungsrichtung ist es vorteilhaft, wenn diese Radialebene mit einer die Rotations-Symmetrieachse der Pfanne enthaltenden Ebene einen Winkel von 20 - 30$^{\circ}$, insbesondere von 25$^{\circ}$, bildet. Eine durch die Knochenschraube praktisch ungestört bleibende Gleitfläche der Pfannenschale erhält man, wenn die Stirnfläche des Schraubenkopfes der Knochenschraube als Teilausschnitt der den Gelenkkopf aufnehmenden Pfannenschale geformt ist, wobei gegebenenfalls unmittelbar nach der Implantation vorhandene geringfügige Niveaudifferenzen durch Fliessen des Materials von Pfanne bzw. Knochenschraube, die beide in erster Linie aus Kunststoff, insbesondere aus Polyäthylen, bestehen, sehr rasch ausgeglichen werden.

Im folgenden wird die Erfindung anhand eines Ausführungsbeispiels im Zusammenhang mit der Zeichnung näher erläutert.

Fig. 1 ist eine Ansicht der neuen Hüftgelenkspfanne in Richtung senkrecht zu der dem Betrachter zugewandten Radialebene für die Zapfenachse;

Fig. 2 gibt eine Aufsicht auf Fig. 1 von oben;

Fig. 3 ist eine Ansicht der Knochenschraube, während

Fig. 4 schliesslich in einem Schnitt IV-IV von Fig. 1 die in ein Becken eingesetzte Pfanne darstellt.

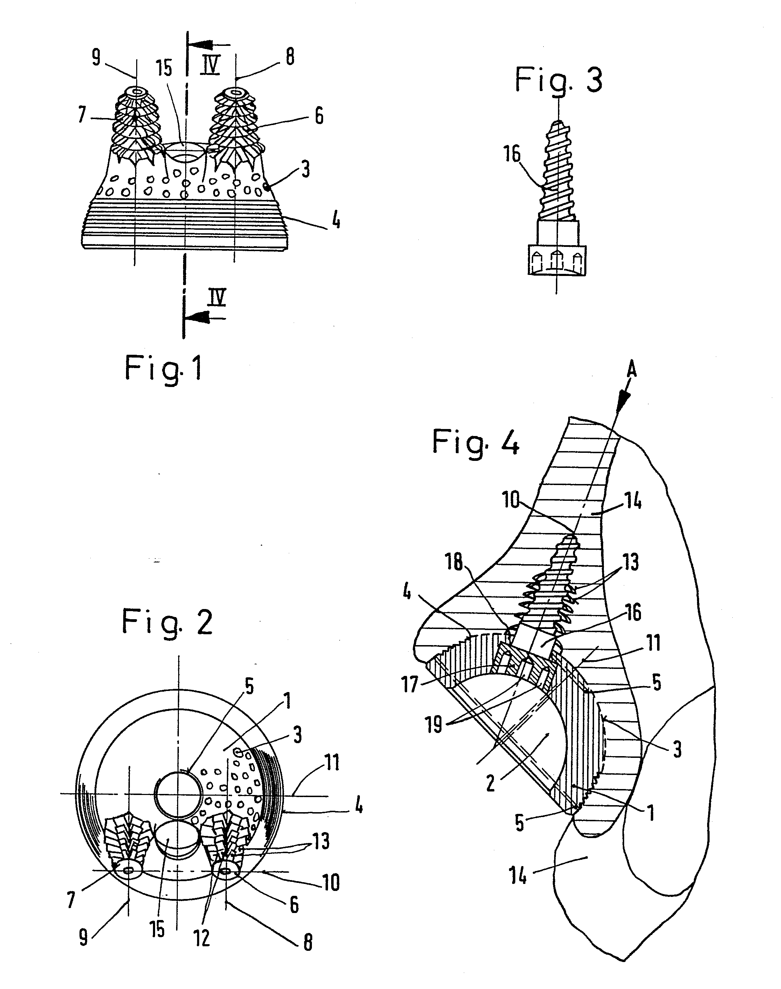

Die in ihrer Aussenform halbkugelige Hüftgelenkspfanne 1 weist konzentrisch mit der Aussenform eine Pfannenschale 2 zur Aufnahme eines nicht gezeigten Gelenkkopfes einer Femurkopfprothese auf (Fig. 4). Ihre Aussenfläche ist von dem Basisdurchmesser her gesehen zunächst mit mehreren zirkularen Verankerungsrippen 4 versehen, an die sich unregelmässig verteilte abgerundete zottenartige Vertiefungen 3, in die Gewebe einwachsen kann, anschliessen. Im Zenit der Halbkugel ist zur Markierung der im allgemeinen aus Kunststoff gefertigten Pfanne 1 in einer Röntgenaufnahme ein Markierungsring 5 (Fig. 2) in die Kugeloberfläche eingelassen.

Aus der Oberfläche der Pfannenhalbkugel 1 ragen zwei Zapfen 6 und 7 heraus, deren Achsen 8 und 9 in einer Radialebene 10 der Pfannenschale 2 liegen. Diese Ebene 10 bildet mit der die Rotationsachse der Pfanne 1 enthaltenden, in Fig. 1 in der Darstellungsebene liegenden Mittelebene 11 (Fig. 4) einen Winkel von 25$^{\circ}$, wodurch bei eingebauter Pfanne 1 - wie Fig. 4 erkennen lässt - die Radialebene 10 in Richtung der durch einen Pfeil A angedeuteten Hauptbelastungsrichtung verläuft.

Die Mantelflächen der leicht konischen Zapfen 6 und 7 sind mit zirkularen, durch Einschnitte 12 in einzelne Lappen 13 unterteilten Verankerungsrippen bedeckt, die nach dem Einschlagen der Pfanne 1 in den Beckenknochen 14 (Fig. 4) Widerlager gegen ein Herauspressen der Zapfen 6 und 7 und damit der Pfanne 1 aus dem Knochen 14 bilden.

Zwischen den Zapfen 6 und 7 ist mit ihrer Achse, ebenfalls in der Radialebene 10 liegend, eine Bohrung 15 für die Aufnahme einer Knochenschraube 16 (Fig. 3) vorgesehen. Diese besitzt die Form konventioneller Knochenschrauben; sie liegt bei eingebauter Pfanne 1 mit ihrem Kopf 17 auf einem Absatz 18 (Fig. 4) der Bohrung 15 auf. Die Stirnfläche der Schraube 16 ist als Teilausschnitt der Pfannenschalenoberfläche ausgebildet, um deren Wirkung als Gleitfläche für den Gelenkkopf nicht zu beeinträchtigen. In diese Stirnfläche sind Bohrungen 19 eingelassen, die zur Aufnahme eines Schraubwerkzeugs dienen.

Patentansprüche

1. Künstliche Hüftgelenkspfanne zur zementfreien Verankerung im Beckenknochen, mit einem kugelschalenförmigen Pfannenkörper, aus dem mit Verankerungsrippen versehene Zapfen hervorspringen, dadurch gekennzeichnet, dass die durch die Achsen (8, 9) der Zapfen (6, 7) definierte Ebene mindestens im wesentlichen eine Radialebene (10) der Pfannenschale (2) ist, und dass in dieser Ebene (10) zwischen den Zapfen durch den kugelschalenförmigen Pfannenkörper eine Bohrung (15) zur Aufnahme einer Knochenschraube (16) führt, wobei die Achse der Bohrung (15) zumindest annähernd ebenfalls in der Radialebene (10) der Zapfenachse (8, 9) liegt, ferner gekennzeichnet durch eine Knochenschraube (16) zur Fixierung der Pfanne (1) im Beckenknochen (14).

2. Hüftgelenkspfanne nach Anspruch 1, dadurch gekennzeichnet, dass die Stirnfläche des Schraubenkopfes (17) der Knochenschraube (16) als Teilausschnitt der den Gelenkkopf aufnehmenden Pfannenschale (2) geformt ist.

3. Hüftgelenkspfanne nach Anspruch 1, dadurch gekennzeichnet, dass die Ebene (10) der Zapfenachsen (8, 9) mit einer die Rotations-Symmetrieachse der Pfanne (1) enthaltenden Mittelebene (11) einen Winkel von $20 - 30^{\circ}$, insbesondere von $25^{\circ}$, bildet.

0121002

Fig. 1

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-3 781 918 (MATHYS)<br>* Spalte 1, Zeile 55 - Spalte 2, Zeile 48; Abbildungen * | 1 | A 61 F 1/03 |
| A | DE-A-2 318 459 (FELDMÜHLE ANLAGEN- UND PRODUKTIONSGESELLSCHAFT)<br>* Abbildung 1; Seite 8, Zeile 5 - Seite 9, Zeile 4 * | 1,2 | |
| D,A | FR-A-1 552 585 (NAT. RESEARCH DEVELOPMENT CORP.) | | |
| D,A | FR-A-2 301 217 (CERAVER) | | |

-----

RECHERCHIERTE SACHGEBIETE (Int. Cl. ³)

A 61 F

Der vorliegende Recherchenbericht wurde für alle Patentansprüche erstellt.

| Recherchenort | Abschlußdatum der Recherche | Prüfer |
|---|---|---|
| DEN HAAG | 13-06-1984 | STEENBAKKER J. |